# FASCICULE DE BREVET EUROPEEN

(11) **EP 1 898 815 B1**
(45) Date de publication et mention de la délivrance du brevet: **21.08.2013**
(21) Numéro de dépôt: 06764624.0
(22) Date de dépôt: 15.05.2006
(51) Int. Cl.: A61B 17/64, A61B 17/66

(54) **Fixateur externe élastique entre deux portions d'os**
Elastisches externes Fixierungselement, das zwischen zwei Knochensegmenten angeordnet ist
Elastic external fixator disposed between two bone segments

(30) Priorité: 26.05.2005 FR 0505289
(43) Date de publication de la demande: 19.03.2008
(73) Titulaire: Renard, Xavier, 91430 Vauhallan (FR)
(72) Inventeur: PELISSIER, Philippe, 33700 Merignac (FR); RENARD, Xavier, 91430 Vauhallan (FR)
(74) Mandataire: Flavenot, Bernard
(86) Numéro de dépôt international: PCT/FR2006/001084
(87) Numéro de publication internationale: WO 2006/125883

(56) Documents cités:
- US-A- 1 997 466
- US-A- 6 162 223

## Description

La présente invention concerne les fixateurs externes élastiques aptes à être montés en coopération avec deux portions d'os entre lesquelles on veut exercer soit une traction soit une distraction, qui trouvent une application particulièrement avantageuse pour traiter, par distraction, des fractures articulaires selon le principe du ligamentotaxis dans lequel la traction exercée de part et d'autre de la fracture réduit le déplacement des fragments et les maintient dans une position susceptible de favoriser le remodelage articulaire.

Il existe déjà de tels fixateurs externes qui trouvent des applications notamment dans le domaine de la réparation osseuse des doigts de la main. Un tel fixateur comprend essentiellement deux crochets aptes à être fixés respectivement sur les deux portions d'os et reliés par un ressort élastique. Ces crochets sont de deux types : de forme rectiligne si l'on veut exercer une traction, en forme sensiblement de "Z" si l'on veut exercer une distraction au moyen d'une force élastique de traction.

Les fixateurs externes connus actuellement ne permettent de moduler l'intensité de la force à exercer entre les deux portions d'os, aussi bien en traction qu'en distraction, et nécessitent au moins deux réalisations différentes selon le type de force que l'on veut exercer.

Le document US 6,162,223 A divulgue un fixateur externe élastique selon le préambule de la revendication 1.

Ces systèmes connus sont essentiellement des montages artisanaux, de réalisation délicate et très dépendants des opérateurs. Les résultats de cette technique connue sont donc aléatoires. Par ailleurs, les montages réalisés sont en général encombrants. Il en est de même des fixateurs externes détournés de leur simple fonction de fixation et rendus mobiles. Leur volume est lié au fait que les systèmes de fixation sur l'os, ceux exerçant la distraction et ceux maintenant l'axe, par exemple du doigt, sont indépendants et occupent chacun un volume propre.

Aussi, la présente invention a-t-elle pour but de réaliser un fixateur externe élastique qui ait une seule structure quel que soit le type de force à exercer entre les deux portions d'os, traction ou distraction, et qui permette de moduler l'intensité de cette force, en autorisant la mobilisation en même temps que la distraction.

Plus précisément, la présente invention a pour objet un fixateur externe élastique entre deux première et seconde portions d'os, comportant :
- un ressort hélicoïdal défini selon un premier axe,
- un premier corps,
- des premiers moyens pour monter en coopération ledit premier corps avec ledit ressort hélicoïdal,
- une première broche apte à être fixée sur ladite première portion d'os, ladite première broche étant de forme oblongue définie selon un deuxième axe,
- des moyens pour monter ladite première broche en coopération avec ledit premier corps de façon que ledit deuxième axe fasse avec le premier axe un angle non nul,
- un second corps, et
- des moyens pour lier ledit second corps avec ladite seconde portion d'os et ledit ressort hélicoïdal,
   caractérisé en ce que ce dit ressort hélicoïdal est apte à pivoter par rapport audit premier corps autour du premier axe et que la première broche traverse ledit ressort hélicoïdal afin de permettre de moduler l'intensité de la force à exercer entre les deux portions d'os.

D'autres caractéristiques et avantages de l'invention apparaîtront au cours de la description suivante donnée en regard des dessins annexés à titre illustratif mais nullement limitatif, dans lesquels :
Les figures 1 et 2 représentent deux vues schématiques orthogonales d'un premier mode de réalisation du fixateur externe selon l'invention,
La figure 3 représente une vue schématique d'un second mode de réalisation du fixateur externe selon l'invention,
La figure 4 représente, sous forme schématique, un autre mode de réalisation d'une partie du fixateur externe selon le mode de réalisation illustré sur la figure 3, à savoir la partie concernant le montage d'une extrémité du ressort hélicoïdal en coopération avec un corps, et
Les figures 5 et 6 représentent, sous forme schématique, deux autres modes de réalisation perfectionnés d'un fixateur externe selon l'invention.

Il est tout d'abord précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est aussi précisé que les figures représentent essentiellement quatre modes de réalisation de l'objet selon l'invention, mais qu'il peut exister d'autres modes de réalisation qui répondent à la définition de cette invention.

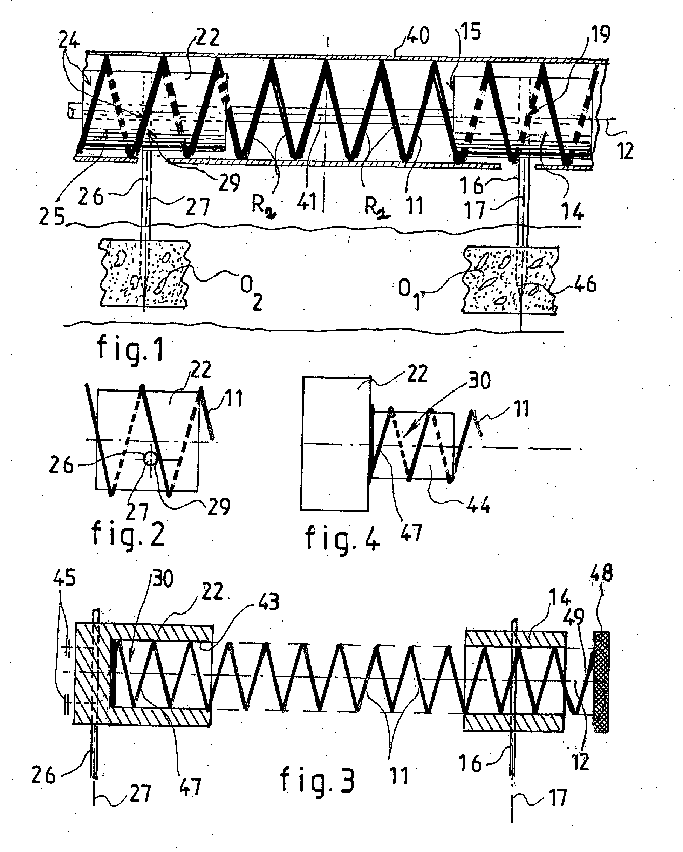

En référence aux figures 1 à 6, l'invention décrite ci-dessous est relative à un fixateur externe apte à exercer une force élastique réglable en intensité entre deux première et seconde portions d'os O₁, O₂, qui trouve une application particulièrement avantageuse comme fixateur externe pour exercer une traction ou une distraction, toutes deux modulables, entre deux os ou deux fractions d'os d'un doigt de la main, par exemple entre deux phalanges.

Selon tous les modes de réalisation illustrés sur les figures 1 à 4, le fixateur externe comporte essentiellement, dans sa définition première, un ressort hélicoïdal 11 défini selon un premier axe 12, un premier corps 14, des premiers moyens 15 pour monter en coopération ce premier corps 14 avec le ressort hélicoïdal 11 de façon que ce ressort hélicoïdal soit apte à pivoter par rapport au premier corps sensiblement autour du premier axe 12, une première broche 16 apte à être fixée sur la première portion d'os O₁, cette première broche 16 étant de forme oblongue définie selon un deuxième axe 17, et des moyens 19 pour monter la première broche 16 en coopération avec le premier corps 14 de façon qu'elle traverse le ressort hélicoïdal 11 et que le deuxième axe 17 fasse avec le premier axe 12 un angle non nul.

Selon une réalisation avantageuse, la broche 16 est une broche osseuse bien connue en elle-même, de forme cylindrique de révolution ou analogue et dont l'extrémité 46 destinée à pénétrer dans l'os comporte un filetage osseux par exemple de type auto taraudant.

Quant aux moyens 19 pour monter cette première broche 16 en coopération avec le premier corps 14, ils sont par exemple constitués d'un orifice traversant réalisé dans le corps 14 et dont la section transversale est complémentaire de celle de la broche 16, et avantageusement de moyens pour bloquer la broche dans l'orifice traversant et donc par rapport au premier corps 14, du genre vis de fixation ou analogue, schématiquement représenté à titre illustratif en 45 sur la figure 3 dans l'application à la fixation de la broche 26 sur le second corps 22 comme il sera décrit ci-après.

Le fixateur externe selon l'invention comporte en outre un second corps 22 et des moyens 24 pour lier ce second corps 22 avec la seconde portion d'os O₂ et le ressort hélicoïdal 11.

Dans le mode de réalisation illustré sur les figures 1 et 2, les moyens 24 pour lier le second corps 22 avec la seconde portion d'os O₂ et le ressort hélicoïdal 11 comportent des seconds moyens 25 pour monter en coopération le second corps 22 avec le ressort hélicoïdal 11 de façon que ce dernier soit apte à pivoter par rapport au second corps autour du premier axe 12, une seconde broche 26 apte à être fixée sur la seconde portion d'os O₂ et définie selon un troisième axe 27, et des moyens 29 pour monter cette seconde broche 26 en coopération avec le second corps 22 de façon qu'elle traverse le ressort hélicoïdal 11 et que le troisième axe 27 fasse avec le premier axe 12 un angle non nul.

Les figures 1 et 2 représentent en fait un premier mode de réalisation du fixateur externe selon l'invention dans lequel les deux corps 14 et 22 sont sensiblement identiques et avantageusement cylindriques de révolution, et les deux broches 16 et 26 traversent le ressort hélicoïdal 11 et respectivement les deux corps 14 et 22 en coopérant de la même façon avec ces deux corps et le ressort hélicoïdal.

Dans une réalisation avantageuse, le ressort hélicoïdal 11 est à spires non jointives et il est réalisé en un matériau comme de l'acier inoxydable ou analogue.

Les premiers et seconds moyens 15, 25 définis ci-dessus pour monter en coopération respectivement les corps 14, 22 avec le ressort hélicoïdal 11 de façon que ce dernier soit apte à pivoter par rapport aux corps autour du premier axe 12, sont constitués par le fait que le ressort hélicoïdal 11 est monté sur les corps selon l'une des deux positions suivantes : une première position dans laquelle il entoure le corps, comme illustré sur la figure 1, ou une seconde position dans laquelle il traverse le corps dans une percée axiale, comme illustré sur la figure 3.

Il est précisé que, selon cette caractéristique de l'invention, le ressort hélicoïdal peut être monté sur les deux corps 14, 22 dans la même position, par exemple en les entourant tous les deux comme illustré sur la figure 1 pour le montage sur les deux corps 14 et 22, ou bien sur l'un des deux corps dans l'une des deux positions, par exemple en le traversant dans une percée axiale comme illustré sur la figure 3 pour le montage sur le premier corps 14, et sur l'autre corps dans l'autre position, c'est-à-dire, dans l'exemple donné ci-avant, en l'entourant comme illustré sur la figure 1 pour le montage sur le second corps 22.

Dans le mode de réalisation en accord avec la figure 1, le ressort hélicoïdal 11 à spires non jointives, comme défini auparavant, comporte deux parties consécutives R₁, R₂, l'enroulement des spires de l'une des deux parties étant en sens inverse de l'enroulement des spires de l'autre partie.

Par référence à la figure 2, on constate que les spires du ressort 11 sont équivalentes à un filetage femelle à pas variable, la broche, par exemple 26, étant équivalente à un point de filet complémentaire mâle qui est apte à suivre la "rampe" des filets du filetage femelle. De cette façon, en faisant pivoter le ressort 11 autour de son axe 12, il se produit un déplacement relatif en rotation et translation du ressort par rapport à la broche 26 en supposant que la broche est fixe. Par référence à la réalisation de la figure 1, les deux parties de ressort R₁ et R₂ ayant des sens d'enroulement inverses, les deux broches 16, 26 vont soit se rapprocher soit s'éloigner l'une de l'autre selon le sens de rotation donné au ressort 11. Mais, si l'on considère que les deux broches sont fixes, le nombre de spires du ressort comprises entre elles va soit augmenter soit diminuer, ce qui entraîne une variation de l'intensité de l'effort de tension exercée par cette partie de ressort entre les deux broches.

La réalisation décrite ci-dessus et illustrée sur les figures 1 et 2 permet de régler l'effort de traction ou de distraction exercé entre les deux portions d'os O₁ et O₂, selon le sens dans lequel est pivoté le ressort hélicoïdal 11 autour de son axe 12 par rapport aux deux corps 14, 22, comme il sera explicité ci-après.

La figure 3 représente un autre mode de réalisation du fixateur externe selon l'invention.

Selon cet autre mode de réalisation, les spires non jointives du ressort hélicoïdal 11 s'enroulent toutes dans le même sens et les premier et second corps 14, 22 sont montés en entourant le ressort.

Le fixateur comporte, comme dans le premier mode de réalisation selon les figures 1 et 2, une première broche osseuse 16 et des moyens 19 pour monter cette première broche 16 en coopération avec le premier corps 14, cette première broche 16 et ces moyens 19 étant comme ceux décrits ci-dessus dans le premier mode de réalisation.

Quant aux moyens 24 pour lier le second corps 22 avec la seconde portion d'os O₂ et le ressort hélicoïdal 11, ils comportent des moyens 30 pour monter une extrémité 47 du ressort hélicoïdal 11 en rotation dans un plan par rapport au second corps 22 et des moyens pour fixer ce second corps avec la seconde portion d'os O₂, ces derniers moyens étant constitués, comme dans le premier mode de réalisation du fixateur, d'une seconde broche 26 qui est à la fois solidaire du second corps 22 et de la seconde portion d'os O₂.

Deux modes de réalisation des moyens 30 définis ci-dessus sont respectivement illustrés sur les figures 3 et 4.

Dans le mode de réalisation selon la figure 3, ces moyens 30 sont constitués par le fait que l'extrémité 47 du ressort hélicoïdal 11 est montée dans un logement en creux 43 réalisé dans le second corps 22. Le ressort peut ainsi pivoter par rapport au second corps, encore plus facilement lorsqu'il est pivoté dans le sens de sa compression, c'est-à-dire lorsqu'il agit en traction sur les deux portions d'os O₁ et O₂ puisque, dans ce cas, son diamètre extérieur diminue.

Dans le mode de réalisation selon la figure 4, ces moyens 30 sont constitués par le fait que l'extrémité 47 du ressort 11 est montée autour d'une partie en saillie 44 solidaire du second corps 22. Le ressort peut ainsi pivoter par rapport au second corps, encore plus facilement quand il est pivoté dans le sens de sa décompression, c'est-à-dire lorsqu'il agit en distraction sur les deux portions d'os O₁ et O₂ puisque, dans ce cas, son diamètre extérieur augmente.

Le fixateur externe peut comporter en outre des moyens de préhension 48 qui sont montés en association avec, par exemple, une extrémité 49 du ressort hélicoïdal 11 pour favoriser son pivotement autour.de son axe 12. Ces moyens de préhension sont par exemple constitués, comme illustré sur la figure 3, par une poignée 48 ou analogue avantageusement moletée ou analogue.

Dans le but de faciliter la réalisation industrielle du fixateur externe selon l'invention, ainsi que sa mise en place et son fonctionnement, il est avantageux que les deuxième et troisième axes 17, 27, comme illustré sur toutes les figures, soient parallèles entre eux et perpendiculaires au premier axe 12.

Le fixateur externe peut comporter en outre une gaine de protection 40 rigide ou élastique, figure 1, entourant extérieurement le ressort hélicoïdal 11, et/ou une tige longitudinale rigide 41 passant à travers au moins l'un des deux corps 14 et 22, en étant par exemple fixée à l'autre, par exemple le corps 14 sur la figure 1, et coulissant dans une percée complémentaire réalisée dans l'autre corps 22.

Les moyens décrits ci-dessus permettent aussi de maintenir le ressort sensiblement rectiligne suivant son axe 12 et de l'empêcher de subir des déformations en forme d'arc quand il exerce un couple de forces, soit en allongement soit en compression, sur les deux corps 14 et 22.

Le fixateur externe selon l'invention fonctionne et s'utilise de la façon suivante:

La description du fonctionnement et de l'utilisation du fixateur externe sera faite tout d'abord en référence au mode de réalisation illustré sur la figure 1.

Il est supposé que des forces de traction ou de distraction doivent être exercées sur les deux portions d'os O₁ et O₂. Pour ce faire, les deux corps 14, 22 sont glissés dans le ressort 11 et positionnés de part et d'autre du changement d'orientation de l'enroulement des spires R1, R2. Les deux corps sont amenés en regard des deux portions d'os O₁ et O₂. Les deux broches 16, 26 sont positionnées respectivement dans les deux corps en traversant le ressort comme décrit ci-avant et leur extrémité filetée est vissée dans la portion d'os correspondante.

Au moyen, par exemple, de vis pointeaux, elles sont ensuite avantageusement solidarisées avec leur corps respectif. On peut aussi supposer que l'un des corps 14, 22 (ou les deux) soit uniquement monté coulissant sur une broche 16, 26, cette broche pouvant avantageusement comporter, par exemple à son extrémité non filetée, une butée ou analogue pour éviter que le corps ne s'échappe de sa broche.

Le ressort est en position de repos et la partie du ressort, comprise entre les deux broches 16, 26, comporte un certain nombre de spires, à savoir sept sur la figure 1.

Si l'on veut exercer une traction entre les deux portions d'os O₁ et O₂, on pivote le ressort autour de son axe 12 de façon que le nombre de spires entre les deux broches diminue.

De cette façon, la portion de ressort comprise entre les deux broches s'étire et exerce sur les broches deux forces de traction opposées qui s'appliquent, via ces broches, sur les deux portions d'os. En pivotant plus ou moins le ressort autour de son axe 12, il est ainsi possible de moduler l'intensité de la traction exercée entre les deux portions d'os.

Si l'on veut exercer une distraction entre les deux portions d'os O1 et O2, on pivote le ressort autour de son axe 12 de façon à augmenter le nombre de spires entre les deux broches. De cette façon, la portion de ressort comprise entre les deux broches se comprime et exerce sur les broches deux forces de distraction opposées qui s'appliquent, via ces broches, sur les deux portions d'os. En pivotant plus ou moins le ressort autour de son axe 12, il est possible de moduler l'intensité de la distraction exercée entre les deux portions d'os.

Avec le mode de réalisation selon les figures 3 et 4, il est aussi possible de réaliser une traction ou une distraction entre les deux portions d'os. Cependant, si l'on veut exercer une traction, il est nécessaire de monter les deux corps 14, 22 et les broches 16, 26 en association avec respectivement les deux portions d'os 01 et 02 de façon que la partie de ressort comprise entre les deux broches soit initialement étirée de façon relativement importante. Le fixateur exerce ainsi sur les deux portions d'os une force de traction plus importante que celle souhaitée mais, en pivotant le ressort de façon à faire augmenter le nombre de spires entre les deux broches, il est possible d'ajuster cette traction.

Si l'on veut exercer une distraction entre les deux portions d'os 01 et 02, il est nécessaire de monter les deux corps 14, 22 et les broches 16, 26 en association avec respectivement ces deux portions d'os de façon que la partie de ressort comprise entre les deux broches soit initialement comprimée de façon relativement importante. Le fixateur exerce ainsi sur les deux portions d'os une force de distraction plus importante que celle souhaitée mais, en pivotant le ressort de façon à faire augmenter le nombre de spires entre les deux broches, il est possible d'ajuster cette distraction.

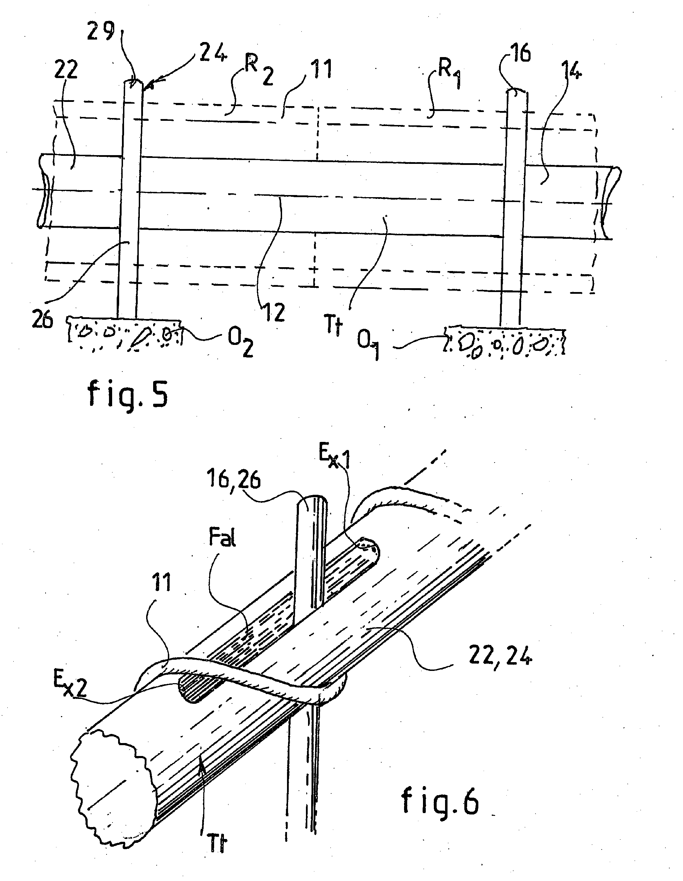

Les figures 5 et 6 représentent deux autres modes de réalisation du fixateur selon l'invention qui sont des variantes équivalentes à celles qui sont décrites ci-avant en regard des figures 1 à 4.

Notamment, la figure 5 représente une variante du fixateur selon laquelle le premier corps 14 et le second corps 22 sont réalisés directement dans une seule et même tige Tt rigide ou souple, et le ressort 11 est formé de la même façon que celui qui est illustré sur la figure 1, c'est-à-dire avec deux enroulements consécutifs en sens contraires R₁ et R₂.

Quant aux moyens 19, 29 pour monter les première et seconde broches 16, 26 en coopération avec la tige Tt, ils sont dans ce cas constitués par le fait que chaque broche passe entre une spire et la tige Tt. Bien entendu, les deux broches passent, l'une 16 dans la partie de ressort R₁ et l'autre 26 dans la partie de ressort R₂. Selon cette réalisation, les deux broches se déplacent en longeant la tige Tt en frottant contre sa paroi latérale, selon que l'on tourne le ressort 11 dans un sens ou dans l'autre comme décrit ci-dessus.

La figure 6 représente une autre variante du fixateur selon l'invention, qui peut être en fait un perfectionnement de la variante selon la figure 5.

En effet, comme mentionné ci-dessus, dans le mode de réalisation selon cette figure 5, quand on fait pivoter le ressort 11, les deux broches peuvent se déplacer par rapport à la tige Tt sans limite. Pour limiter l'amplitude de la traction ou de la distraction entre les deux portions d'os O₁, O₂, au moins l'une des deux broches 14, 16 passe dans une fente oblongue Fal réalisée dans la tige Tt suivant son axe longitudinal 12, comme représenté sur la figure 6.
Dans ce cas, l'amplitude de la traction ou de la distraction est limitée par le fait que la broche 14 ou 16 vient buter contre l'une ou l'autre des extrémités de fond Ex1, Ex2 de la fente oblongue Fal.

## Revendications

1. Fixateur externe élastique entre deux première et seconde portions d'os (O₁, O₂), comportant:
• un ressort hélicoïdal (11) défini selon un premier axe (12),
• un premier corps (14),
• des premiers moyens (15) pour monter en coopération ledit premier corps (14) avec ledit ressort hélicoïdal (11) ,
• une première broche (16) apte à être fixée sur ladite première portion d'os (O₁), ladite première broche (16) étant de forme oblongue définie selon un deuxième axe (17),
• des moyens (19) pour monter ladite première broche (16) en coopération avec ledit premier corps (14) de façon que ledit deuxième axe (17) fasse avec le premier axe (12) un angle non nul,
• un second corps (22), et
• des moyens (24) pour lier ledit second corps (22) avec ladite seconde portion d'os (O₂) et ledit ressort hélicoïdal (11),
**caractérisé en ce que** ce dit ressort hélicoïdal est apte à pivoter par rapport audit premier corps autour du premier axe (12), et que la première broche traverse ledit ressort hélicoïdal (11) afin de permettre de moduler l'intensité de la force à exercer entre les deux portions d'os.

2. Fixateur selon la revendication 1, **caractérisé par le fait que** les moyens (24) pour lier ledit second corps (22) avec ladite seconde portion d'os (02) et ledit ressort hélicoïdal (11) comportent :
• des seconds moyens (25) pour monter en coopération ledit second corps (22) avec ledit ressort hélicoïdal (11) de façon que ce dit ressort soit apte à pivoter par rapport audit second corps autour du premier axe (12),
• une seconde broche (26) apte à être fixée sur ladite seconde portion d'os, ladite seconde broche étant de forme oblongue définie selon un troisième axe (27), et
• des moyens (29) pour monter ladite seconde broche en coopération avec ledit second corps de façon qu'elle traverse ledit ressort hélicoïdal (11) et que ledit troisième axe (27) fasse avec le premier axe (12) un angle non nul.

3. Fixateur selon la revendication 2, **caractérisé par le fait que** ledit ressort hélicoïdal (11) est à spires non jointives.

4. Fixateur selon la revendication 3, **caractérisé par le fait que** ledit ressort hélicoïdal (11) comporte deux parties consécutives (R₁, R₂), l'enroulement des spires de l'une des deux parties étant en sens inverse de l'enroulement des spires de l'autre partie.

5. Fixateur selon l'une des revendications 2 à 4, **caractérisé par le fait que** les premiers et seconds moyens (15, 25) pour monter en coopération les corps (14, 22) avec ledit ressort hélicoïdal (11) de façon que ce dit ressort hélicoïdal soit apte à pivoter par rapport à ces corps autour du premier axe (12), sont constitués par **le fait que** ledit ressort hélicoïdal (11) est monté sur ces corps selon l'une des deux positions suivantes : une première position dans laquelle il traverse ledit corps dans une percée axiale, une seconde position dans laquelle il entoure ledit corps.

6. Fixateur selon la revendication 1, **caractérisé par le fait que** les moyens (24) pour lier ledit second corps (22) avec ladite seconde portion d'os (O₂) et ledit ressort hélicoïdal (11) comportent des moyens (30) pour monter une extrémité (47) du dit ressort hélicoïdal (11) en rotation dans un plan par rapport au dit second corps (22) et des moyens pour fixer ledit second corps avec ladite seconde portion d'os (O₂).

7. Fixateur selon l'une des revendications précédentes, **caractérisé par le fait que** ledit ressort hélicoïdal agit selon l'une des deux façons suivantes : en traction, en distraction.

8. Fixateur selon l'une des revendications précédentes, **caractérisé par le fait qu'**il comporte des moyens de préhension montés en association avec ledit ressort hélicoïdal pour le faire pivoter autour du premier axe (12).

9. Fixateur selon l'une des revendications 2 à 8, **caractérisé par le fait que** les deuxième et troisième axes (17, 27) respectivement des première et seconde broche (16, 26) sont parallèles entre eux et perpendiculaires au premier axe (12).

10. Fixateur selon l'une des revendications précédentes, **caractérisé par le fait qu'**il constitue, à titre d'application, un fixateur externe entre deux points osseux d'un doigt d'une main d'un être humain.

11. Fixateur selon l'une des revendications précédentes, **caractérisé par le fait qu'**il comporte des moyens pour maintenir ledit ressort (11) sensiblement rectiligne suivant son axe (12).

12. Fixateur selon la revendication 4 et l'une des revendications 5 à 11 quand elle dépend de la revendication 4, **caractérisé par le fait que** les deux corps (14, 22) sont réalisés dans une même tige (Tt).

13. Fixateur selon la revendication 12, **caractérisé par le fait qu'**au moins l'une des deux broches (14, 26) traverse ladite tige dans une fente oblongue (Fal) réalisée suivant l'axe longitudinal (12) de ladite tige (Tt).

## Patentansprüche

1. Elastische externe Befestigungseinrichtung zwischen zwei Knochenabschnitten (O₁, O₂), nämlich einem ersten und einem zweiten Knochenabschnitt (O₁, O₂), die umfasst:
- eine Schraubenfeder (11), die längs einer ersten Achse (12) definiert ist,
- einen ersten Körper (14),
- erste Mittel (15), um den ersten Körper (14) in Zusammenwirkung mit der Schraubenfeder (11) zu montieren,
- einen ersten Stift (16), der an dem ersten Knochenabschnitt (O₁) befestigt werden kann, wobei der erste Stift (16) eine längliche Form hat, die längs einer zweiten Achse (17) definiert ist,
- Mittel (19), um den ersten Stift (16) in Zusammenwirkung mit dem ersten Körper (14) so zu montieren, dass die zweite Achse (17) mit der ersten Achse (12) einen von null verschiedenen Winkel bildet,
- einen zweiten Körper (22) und
- Mittel (24), um den zweiten Körper (22) mit dem zweiten Knochenabschnitt (O₂) und der Schraubenfeder (11) zu verbinden,
**dadurch gekennzeichnet, dass** die Schraubenfeder in Bezug auf den ersten Körper um die erste Achse (12) schwenken kann und dass der erste Stift durch die Schraubenfeder (11) verläuft, um die Modulation der Stärke der Kraft, die zwischen den zwei Knochenabschnitten auszuüben ist, zu ermöglichen.

2. Befestigungseinrichtung nach Anspruch 1, **dadurch gekennzeichnet, dass** die Mittel (24) zum Verbinden des zweiten Körpers (22) mit dem zweiten Knochenabschnitt (O₂) und der Schraubenfeder (11) umfassen:
- zweite Mittel (25), um den zweiten Körper (22) in Zusammenwirkung mit der Schraubenfeder (11) zu montieren, derart, dass diese Feder in Bezug auf den zweiten Körper um die erste Achse (12) schwenken kann,
- einen zweiten Stift (26), der an dem zweiten Knochenabschnitt befestigt werden kann, wobei der zweite Stift eine längliche Form besitzt, die längs einer dritten Achse (27) definiert ist, und
- Mittel (29), um den zweiten Stift in Zusammenwirkung mit dem zweiten Körper zu montieren, derart, dass er durch die Schraubenfeder (11) verläuft und dass die dritte Achse (27) mit der ersten Achse (12) einen von null verschiedenen Winkel bildet.

3. Befestigungseinrichtung nach Anspruch 2, **dadurch gekennzeichnet, dass** die Schraubenfeder (11) vom Typ mit nicht benachbarten Windungen ist.

4. Befestigungseinrichtung nach Anspruch 3, **dadurch gekennzeichnet, dass** die Schraubenfeder (11) zwei aufeinander folgende Teile (R₁, R₂) aufweist, wobei die Wicklung der Windungen eines der zwei Teile einen Richtungssinn hat, die zu jenem der Wicklung der Windungen des anderen Teils entgegengesetzt ist.

5. Befestigungseinrichtung nach einem der Ansprüche 2 bis 4, **dadurch gekennzeichnet, dass** die ersten und zweiten Mittel (15, 25) zum Montieren der Körper (14, 22) in Zusammenwirkung mit der Schraubenfeder (11), derart, dass diese Schraubenfeder in Bezug auf diese Körper um die erste Achse (12) schwenken kann, durch die Tatsache gebildet sind, dass die Schraubenfeder (11) an diesen Körpern längs einer der zwei folgenden Positionen montiert ist: einer ersten Position, in der sie durch den Körper in einem axialen Loch verläuft, und einer zweiten Position, in der sie den Körper umgibt.

6. Befestigungseinrichtung nach Anspruch 1, **dadurch gekennzeichnet, dass** die Mittel (24) zum Verbinden des zweiten Körpers (22) mit dem zweiten Knochenabschnitt (O₂) und der Schraubenfeder (11) Mittel (30), um ein Ende (47) der Schraubenfeder (11) drehbar in einer Ebene in Bezug auf den zweiten Körper (22) zu montieren, und Mittel, um den zweiten Körper an dem zweiten Knochenabschnitt (O₂) zu befestigen, umfassen.

7. Befestigungseinrichtung nach einem der vorhergehenden Anspruche, **dadurch gekennzeichnet, dass** die Schraubenfeder auf eine der zwei folgenden Weisen wirkt: Traktion, Distraktion.

8. Befestigungseinrichtung nach einem der vorhergehenden Ansprüche, **dadurch gekennzeichnet, dass** sie Greifmittel umfasst, die in Zuordnung zu der Schraubenfeder montiert sind, um sie um die erste Achse (12) zu schwenken.

9. Befestigungseinrichtung nach einem der Ansprüche 2 bis 8, **dadurch gekennzeichnet, dass** die zweite und dritte Achse (17, 27) des ersten bzw. des zweiten Stifts (16, 26) zueinander parallel und zu der ersten Achse (12) senkrecht sind.

10. Befestigungseinrichtung nach einem der vorhergehenden Ansprüche, **dadurch gekennzeichnet, dass** sie bei ihrer Anwendung eine externe Befestigungseinrichtung zwischen zwei Knochenpunkten eines Fingers einer Hand eines menschlichen Wesens bildet.

11. Befestigungseinrichtung nach einem der vorhergehenden Ansprüche, **dadurch gekennzeichnet, dass** sie Mittel umfasst, um die Feder (11) im Wesentlichen geradlinig längs ihrer Achse (12) zu halten.

12. Befestigungseinrichtung nach Anspruch 4 und einem der Ansprüche 5 bis 11, wenn abhängig von Anspruch 4, **dadurch gekennzeichnet, dass** die zwei Körper (14, 22) aus demselben Stab (Tt) gebildet sind.

13. Befestigungseinrichtung nach Anspruch 12, **dadurch gekennzeichnet, dass** wenigstens einer der zwei Stifte (14, 26) den Stab in einem länglichen Schlitz (Fal), der längs einer Längsachse (12) des Stabs (Tt) verwirklicht ist, durchquert.

## Claims

1. A resilient external fixator between first and second bone portions (O₁, O₂), comprising:
· a helical spring (11) defined along a first axis (12);
· a first body (14);
· first means (15) for mounting said first body (14) in co-operation with said helical spring (11) ;
· a first pin (16) suitable for being fastened to said first bone portion (O₁), said first pin (16) being oblong in shape and defined along a second axis (17);
· means (19) for mounting said first pin (16) to co-operate with said first body (14) so that said second axis (17) forms a non-zero angle with the first axis (12);
· a second body (22); and
· means (24) for connecting said second body (22) with said first bone portion (O₂) and said helical spring (11),
**characterized by** the fact that said helical spring is suitable for turning relative to said first body about the first axis (12) and that said first pin passes through said helical spring (11) in order to permit the modulation of the intensity of the force to be exerted between the two bone portions.

2. A fixator according to claim 1, **characterized by** the fact that the means (24) for connecting said second body (22) with said second bone portion (O₂) and said helical spring (11) comprise:
· second means (25) for mounting said second body (22) in co-operation with said spring so that said helical spring is suitable for turning relative to said second body about the first axis (12);
· a second pin (26) suitable for being fastened to said second bone portion, said second pin being oblong in shape and defined along a third axis (27); and
· means (29) for mounting said second pin to co-operate with said second body so as to pass through said helical spring (11) and so that said third axis (27) forms a non-zero angle with the first axis (12).

3. A fixator according to claim 2, **characterized by** the fact that said helical spring (11) has non-touching turns.

4. A fixator according to claim 3, **characterized by** the fact that said helical spring (11) comprises two consecutive portions (R₁, R₂), the turns in one of the two portions being wound in the opposite direction to the direction in which the turns in the other portion are wound.

5. A fixator according to any one of claims 2 to 4, **characterized by** the fact that the first and second means (15, 25) for mounting the bodies (14, 22) in co-operation with said helical spring (11) so that said helical spring is suitable for turning relative to said bodies about the first axis (12) are constituted by the fact that said helical spring (11) is mounted on said bodies in one of the following two positions: a first position in which it passes through said body in an axial bore; a second position in which it surrounds said body.

6. A fixator according to claim 1, **characterized by** the fact that the means (24) for connecting said second body (22) with said second bone portion (O₂) and said helical spring (11) comprise means (30) for mounting one end (47) of said helical spring (11) to turn in a plane relative to said second body (22), and means for fastening said second body with said second bone portion (O₂).

7. A fixator according to any preceding claim, **characterized by** the fact that said helical spring acts in one of the following two manners: in traction; in distraction.

8. A fixator according to any preceding claim, **characterized by** the fact that it includes grip means mounted in association with said helical spring to cause it to turn about the first axis (12).

9. A fixator according to any one of claims 2 to 8, **characterized by** the fact that the second and third axes (17, 27) respectively of the first and second pins (16, 26) are mutually parallel and perpendicular to the first axis (12).

10. A fixator according to any preceding claim, **characterized by** the fact that, by way of application, it constitutes an external fixator between two bone points in a finger of a human being.

11. A fixator according to any preceding claim, **characterized by** the fact that it includes means for keeping said spring (11) substantially rectilinear along its axis (12).

12. A fixator according to claim 4 and any one of claims 5 to 11 when dependent on claim 4, **characterized by** the fact that both bodies (14, 22) are formed in a single rod (Tₜ).

13. A fixator according to claim 12, **characterized by** the fact that at least one of the two pins (14, 26) passes through said rod in an oblong slot (Fₐₗ) extending along the longitudinal axis (12) of said rod (Tₜ).
